# FASCICULE DE BREVET EUROPEEN

(11) **EP 1 593 368 B1**
(45) Date de publication et mention de la délivrance du brevet: **01.11.2017**
(21) Numéro de dépôt: 05300325.7
(22) Date de dépôt: 26.04.2005
(51) Int. Cl.: A61K 8/04, A61K 8/31, A61K 8/34, A61Q 5/06, A61K 8/81

(54) **Composition cosmétique exempte d'eau comprenant des esters d'acides gras, des polymères non ioniques et de l'huile de vaseline**
Wasserfreie kosmetische Zusammensetzungen enthaltend Fettsäureester, nicht-ionische Polymere und Vaselinöl
Anhydrous cosmetic compositions comprising fatty acid esters, non-ionic polymers and vaseline oil

(30) Priorité: 27.04.2004 FR 0450805
(43) Date de publication de la demande: 09.11.2005
(73) Titulaire: L'Oréal, 75008 Paris (FR)
(72) Inventeur: Pataut, Françoise, 75017, PARIS (FR); Gringore, Charles, 75017, PARIS (FR)
(74) Mandataire: Caillet, Isabelle

(56) Documents cités:
- EP-A- 1 366 748
- FR-A- 1 102 563
- PATENT ABSTRACTS OF JAPAN vol. 0040, no. 16 (C-072), 7 février 1980 (1980-02-07) & JP 54 151140 A (SHISEIDO CO LTD), 28 novembre 1979 (1979-11-28)

## Description

La présente invention se rapporte à une composition cosmétique exempte d'eau comprenant un polymère fixant non ionique, une huile de vaseline, un alcanol, et un cosolvant, tels que définis dans la revendication 1, pour la mise en forme non permanente de la chevelure.

Plus particulièrement, la présente invention se rapporte à une telle composition cosmétique pour la mise en forme de la chevelure, qui lui apporte également de la brillance sans conduire à un toucher « synthétique ».

Fixer une coiffure tout en lui apportant de la brillance est aujourd'hui une demande de plus en plus importante des consommatrices.

Le type de composition le plus couramment rencontrée repose sur l'association de polymères fixants anioniques ou non ioniques avec des silicones, notamment des silicones phénylées.

Néanmoins ce type d'association peut conduire à un toucher très « synthétique », désagréable en particulier pour certains types de cheveux.

La demande de brevet JP 54151140 divulgue ainsi une composition contenant de l'huile de vaseline, une huile de silicone, des esters d'acide gras, dans une solution eau-alcool comprenant 5% à 20% en eau.

Il est connu que l'huile de vaseline associée à un polymère non ionique apporte de la brillance et du maintien à la chevelure, mais une trop grande concentration déposée induit un toucher très gras.

Une manière pratique et efficace de déposer de l'huile de vaseline sur les cheveux est de la véhiculer via un spray aérosol ou non aérosol permettant ainsi une excellente répartition et un respect de la forme de la coiffure. Par ailleurs, le véhicule doit s'évaporer rapidement pour permettre un dépôt de l'huile sur la chevelure.

Lesdits sprays aérosols ou non-aérosols doivent en outre permettre une pulvérisation très peu sternutatoire.

Les alcanols inférieurs en C₁-C₆, notamment l'éthanol seraient de bons candidats comme véhicules pour de telles compositions contenant de l'huile de vaseline, mais celle-ci n'est pas soluble dans ces alcanols, en particulier l'éthanol.

La présente invention a donc pour but de réaliser des compositions cosmétiques pour la fixation de la coiffure tout en leur apportant de la brillance et sans risque de conduire à un toucher synthétique, permettant une excellente répartition et un respect de la forme, notamment lorsque ces compositions sont sous forme de spray aérosol ou non.

L'invention vise donc de telles compostions comprenant une quantité relativement importante d'huile de vaseline, une faible quantité de silicones, voire pas de silicones du tout, et qui soit exempte d'eau.

Le but de la présente invention est également de réaliser de telles compositions sous forme de sprays, aérosols ou non-aérosols permettant d'apporter de la brillance et du maintien à la chevelure en tout remédiant aux inconvénients de l'art antérieur.

La demanderesse a trouvé de façon inattendue qu'il était possible de remédier aux inconvénients de l'art antérieur et de remplir les objectifs précités en réalisant une composition capillaire exempte d'eau comprenant au moins un polymère fixant non ionique, une huile de vaseline, au moins un alcanol en C₁-C₆ et au moins un ester d'acide gras.

Ainsi, l'invention a pour premier objet une composition cosmétique exempte d'eau caractérisée en ce qu'elle comprend au moins un polymère fixant non ionique, une huile de vaseline, au moins un alcanol inférieur en C₁-C₆, et au moins un ester d'acide gras, la composition étant telle que définie dans la revendication 1. La quantité nécessaire d'huile est ainsi déposée sur la chevelure après évaporation du solvant alcanolique.

Comme indiqué précédemment, la composition selon l'invention comprend au moins un polymère fixant non ionique.

Par polymère fixant on entend au sens de la présente invention tout polymère permettant de conférer une forme à la chevelure ou de modifier la forme de la chevelure.

Les polymères fixants non ioniques utilisables selon la présente invention sont choisis parmi les polymères non ioniques à base de vinylpyrrolidone.

Selon un mode de réalisation préférentiel de l'invention, les polymères fixants non ioniques sont choisis parmi les homopolymères de vinylpyrrolidone ou les copolymères de vinylpyrrolidone et d'acétate de vinyle.

Le ou les polymères fixants non ioniques représentent généralement 0,01 à 8%, de préférence de 0,1 à 3%, en poids par rapport au poids total de la composition.

Typiquement, l'huile de vaseline représente 3 à 20%, de préférence 5 à 10% par rapport au poids total de la composition.

La composition selon l'invention comprend également au moins un alcanol.

L'alcanol convenant pour la composition de la présente invention est l'éthanol.

Le ou les alcanols représentent généralement de 5 à 75%, de préférence de 10 à 60%, mieux de 15 à 50%, en poids par rapport au poids total de la composition.

La composition objet de l'invention comprend également au moins un ester d'acide gras.

Au sens de la présente invention, on entend par ester d'acide gras un ester d'acide carboxylique, ledit acide comportant au moins 8 atomes de carbone. De préférence, l'acide est de formule RCOOH, R étant une chaîne hydrocarbonée saturée ou ramifiée comportant au moins 7 atomes de carbone.

On préfère à titre d'ester d'acide gras selon l'invention les esters d'acides monocarboxyliques aliphatiques linéaires en C₈-C₂₈ saturés ou insaturés, linéaires ou ramifiés et de monoalcools en C₁-C₂₈ saturés ou insaturés, linéaires ou ramifiés.

Selon l'invention, l'ester d'acide gras est un monocarboxylate d'alkyle en C₁-C₂₂ tels que les monocarboxylates de méthyle, de propyle, de butyle, d'isopropyle ou d'hexyle.

Parmi les monocarboxylates d'alkyle en C₁-C₂₂, on peut encore citer le béhénate de dihydroabiétyle, le béhénate d'octyldodécyle, le béhénate d'isocétyle ; l'octanoate de (iso)stéaryle, l'octanoate d'isocétyle, l'octanoate d'octyle, l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle, l'isononanoate d'octyle, l'isononate de 2-éthylhexyle ; le stéarate de myristyle ; le pélargonate d'octyle ; le stéarate d'octyle ; l'érucate d'octyldodécyle, l'érucate d'oléyle ; le palmitate d'octyle, le palmitate d'isostéaryle, les palmitates d'éthyle et d'isopropyle, le palmitate d'éthyl-2-héxyle, le palmitate de 2-octyldécyle, les myristates d'alkyles en C₁-C₁₀, de préférence en C₁-C₆, tels que le myristate d'isopropyle, de butyle, de cétyle ; le stéarate d'hexyle, le stéarate de butyle, le stéarate d'isobutyle ; le laurate d'hexyle, le laurate de 2-hexyldécyle, ou un mélange de ceux-ci

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles en C₁-C₁₀, de préférence en C₁-C₆ tels que le myristate d'isopropyle, de butyle, de cétyle, de 2-octyldodécyle, le stéarate d'hexyle, le stéarate de butyle, le stéarate d'isobutyle ; le laurate d'hexyle, le laurate de 2-hexyldécyle et l'isononanate d'isononyle, l'octanoate de cétyle.

De préférence, on utilisera un ester d'acide gras liquide à la température ambiante de 25°C et à la pression atmosphérique ambiante.

Le ou les esters d'acide gras représentent généralement de 1 à 50%, de préférence de 5 à 35%, mieux de 10 à 30%, en poids par rapport au poids total de la composition.

Dans le but d'améliorer les propriétés cosmétiques des fibres capillaires ou encore d'atténuer ou d'éviter leur dégradation, la composition utilisée selon l'invention peut également comprendre un ou plusieurs additifs cosmétiques.

Ce ou ces additifs sont généralement choisis parmi les agents protecteurs des fibres capillaires, les vitamines ou provitamines, les parfums, les conservateurs, les séquestrants, les agents acidifiants, alcalinisants, ainsi que leurs mélanges.

Le ou les additifs cosmétiques représentent généralement de 0,001 à 20%, de préférence de 0,1 à 5%,en poids par rapport au poids total de la composition.

Bien que de préférence, les compositions selon l'invention ne comprennent pas de silicones, elles peuvent néanmoins en compter en faibles quantités.

Ces silicones peuvent être volatiles ou non, linéaires ou cycliques.

Les silicones utilisables conformément à l'invention sont en particulier des polyorganosiloxanes et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels, ainsi que leurs mélanges.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécane ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut notamment citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (I) : 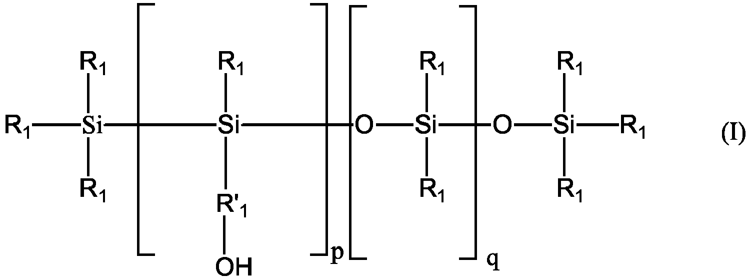 dans laquelle les radicaux R₁ identiques ou différents sont choisis parmi les radicaux méthyle et phényle ; au moins 60 % en mole des radicaux R₁ désignant méthyle ; le radical R'₁ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans le brevet US-A-4957732 et répondant à la formule (II) : 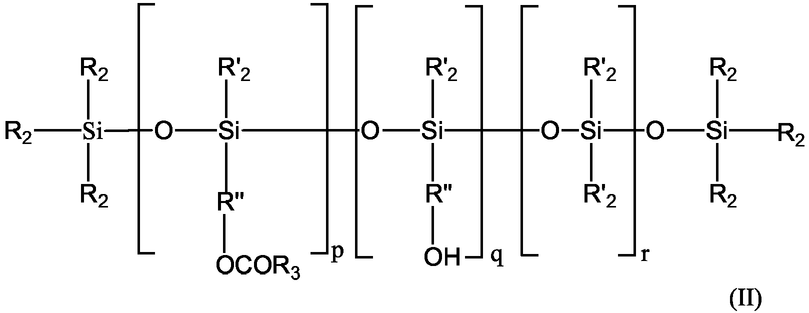 dans laquelle :
   R₂ désigne un groupement méthyle, phényle, -OCOR₃, hydroxyle, un seul des radicaux R₂ par atome de silicium pouvant être OH ;
   R'₂ désigne méthyle, phényle ; au moins 60 % en proportion molaire de l'ensemble des radicaux R₂ et R'₂ désignant méthyle ;
   R₃ désigne alkyle ou alcényle en C₈-C₂₀ ;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (II) peuvent contenir des groupements : 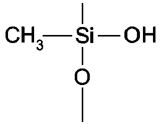 dans des proportions ne dépassant pas 15 % de la somme p + q + r.
      - des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
      - des groupements hydroxyacylamino, comme les polyorganosiloxanes décrits dans la demande EP 342 834. On peut citer par exemple le produit Q2-8413 de la société DOW CORNING.

Les polyorganosiloxanes particulièrement préférés conformément à l'invention sont polyorganosiloxanes comportant des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200.

Selon l'invention, toutes les silicones peuvent également être utilisées sous forme soluble, dispersée, micro dispersée, d'émulsions, de nanoémulsions ou de microémulsions.

En général, la composition selon l'invention comprend moins de 1% de silicone en poids du poids total de la composition.

De manière avantageuse, la composition peut également comprendre un ou plusieurs solvants additionnels cosmétiquement acceptables, choisis de préférence parmi les polyols tels que le glycérol, le propylène glycol, le pentanediol, et l'alcool benzylique.

Le ou les solvants additionnels cosmétiquement acceptables selon l'invention représentent de 0,001 à 5% du poids total de la composition, de préférence de 0,1 à 1% par rapport au poids total de la composition.

Les agents protecteurs des fibres capillaires peuvent être tout agent actif utile pour prévenir ou limiter les dégradations dues aux agressions physiques ou chimiques.

Ainsi, l'agent protecteur des fibres capillaires peut être choisi parmi les filtres UV organiques hydrosolubles, liposolubles ou insolubles dans l'eau, les agents antiradicalaires, les agents antioxydants, les vitamines, les provitamines ainsi que leurs mélanges.

Les filtres UV organiques (systèmes filtrant les radiations UV) sont notamment choisis parmi les filtres hydrosolubles ou liposolubles, siliconés ou non siliconés et les nanoparticules d'oxydes minéraux dont la surface a éventuellement été traitée pour la rendre hydrophile ou hydrophobe.

Les filtres UV organiques hydrosolubles peuvent être choisis parmi par exemple, l'acide para-aminobenzoïque et ses sels, l'acide anthranilique et ses sels, l'acide salicylique et ses sels, l'acide p-hydroxycinnamique et ses sels, les dérivés sulfoniques de benz-x-azole (benzothioazoles, benzimidazoles, benzoxazoles) et leurs sels, les dérivés sulfoniques de la benzophénone et leurs sels, les dérivés sulfoniques de benzylidène camphre et leurs sels, les dérivés de benzylidène camphre substitués par une amine quaternaire et leurs sels, les dérivés des acides phtalydène-camphosulfoniques et leurs sels, les dérivés sulfoniques de benzotriazole.

On peut également utiliser des polymères hydrophiles présentant, en outre et de par leur nature chimique, des propriétés de photoprotection contre le rayonnement UV. On peut citer les polymères comportant des groupements benzylidène camphre et/ou benzotriazole, substitués par des groupements sulfoniques ou ammonium quaternaires.

Comme filtres UV organiques liposolubles (ou lipophiles) convenant à une mise en oeuvre dans la présente invention, on peut notamment citer : les dérivés d'acide p-aminobenzoïque, tels que les esters ou amides d'acide p-aminobenzoïque ; les dérivés d'acide salicylique tels que les esters; les dérivés de benzophénone ; les dérivés de dibenzoylméthane ; les dérivés de diphénylacrylates ; les dérivés de benzofurannes ; les filtres UV polymères contenant un ou plusieurs résidus silico-organiques ; les esters d'acide cinnamique ; les dérivés de camphre ; les dérivés de trianilino-s-triazine ; l'ester éthylique d'acide urocanique ; les benzotriazoles ; les dérivés d'hydroxyphényltriazine ; les bis-résorcinol-dialkylaminotriazine ; et leurs mélanges.

Le filtre UV liposoluble (ou lipophile) selon l'invention est de préférence choisi parmi : l'octyl salicylate ; le 4-tertiobutyl 4'-méthoxydibenzoylméthane (PARSOL 1789 de GIVAUDAN); l'octocrylène ; le 4-méthoxy cinnamate de 2 -éthylhexyl (PARSOL MCX) et le composé de formule (III) suivante, ou 2-(2H-benzotriazole-2-yl)-4-méthyl-6-[2-méthyl-3-[1,3,3,3-tétraméthyl-1-[(triméthylsilyl)oxy] disiloxanyl]propynyl]phénol, décrit dans la demande de brevet EP-A-0 392 883 :

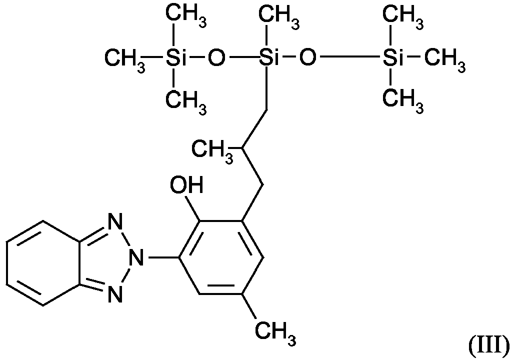

D'autres filtres UV particulièrement préférés selon l'invention sont les dérivés de benzophénones tels que l'UVINUL MS 40 (Acide 2-hydroxy 4-méthoxybenzophénone-5-sulfonique) et l'UVINUL M40 (2-hydroxy-4-méthoxybenzophénone) commercialisés par BASF, les dérivés de benzalmalonates tels que le PARSOL SLX (poly diméthyl/méthyl (3(4-(2,2-bis-éthoxycarbonylvinyl)-phénoxy)-propényl) siloxane) commercialisé par GIVAUDAN-ROURE, les dérivés de benzylidènecamphre tels que le MEXORYL SX (acide b-b'camphosulfonique [1-4 divinylbenzène]) fabriqué par la société CHIMEX, les dérivés de benzimidazole tels que l'EUSOLEX 232 (acide 2-phényl-benzimidazol-5-sulfonique) commercialisé par MERCK.

Le pH peut être ajusté à la valeur désirée au moyen d'agents acidifiants ou alcalinisants bien connus de l'état de la technique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (IV) suivante : 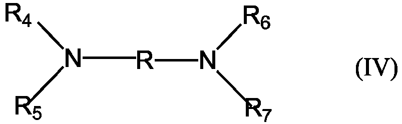 dans laquelle R est un reste propylène éventuellement substitué par un groupement hydroxyle ou un radical alkyle en C₁-C₄ ; R₄, R₅, R₆ et R₇, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition selon la présente invention peut avantageusement être pressurisée en aérosol, et comprendre au moins un agent propulseur, de préférence choisi parmi les alcanes volatils tels que le n-butane, le propane, l'isobutane, le pentane, le gaz carbonique, le protoxyde d'azote, le diméthyléther, l'azote, l'air comprimé ou leurs mélanges.

De préférence, l'agent propulseur choisi est l'isobutane.

Le ou les agents propulseurs représentent généralement 25 à 45%, de préférence de 30 à 40% du mélange total d'agents propulseurs et de la composition telle que décrite ci-dessus.

L'invention a aussi pour objet un procédé de fabrication d'une composition cosmétique comprenant :
a) La dissolution de l'huile de vaseline dans au moins un ester d'acide gras ; et
b) l'addition de la solution obtenue à l'étape a) dans un mélange préalablement formé d'au moins un polymère fixant non ionique et d'au moins un alcanol.

L'invention a également pour objet un procédé comprenant la pressurisation de ladite composition cosmétique dans un récipient au moyen d'un agent de propulsion.

La présente invention a encore pour but de réaliser une composition cosmétique pouvant être utilisée sous forme de spray aérosol comme décrit ci-dessus ou non aérosol par utilisation d'un flacon pompe.

Dans le cas de l'aérosol, la présence d'alcool permet une pulvérisation très peu sternutatoire.

L'utilisation de la composition selon l'invention permet d'apporter de la brillance à la chevelure ainsi traitée, et notamment un toucher naturel.

La présente invention est illustrée par l'exemple suivant :

### EXEMPLE

On réalise un spray aérosol « brillant fixant » contenant en grammes pour 100 grammes:

| | |
|---|---|
| Myristate d'isopropyl : | 26% |
| Huile de vaseline : | 6,5% |
| Silicone OE/OP : | 0,5% |
| Polyvinylpyrrolidone : | 0,65% |
| Ethanol : | 31,35% |
| Isobutane : | 35% |

Silicone OE/OP : Silicone comportant des groupements oxyéthylène et oxypropylène.

Après application sur la coiffure, on constate une brillance et un maintien de la chevelure, sans les inconvénients habituels de toucher gras ou synthétique.

## Revendications

1. Composition cosmétique exempte d'eau **caractérisée en ce qu'**elle comprend au moins un polymère fixant non ionique, une huile de vaseline, au moins un alcanol inférieur en C₁-C₆, et au moins un ester d'acide gras, dans laquelle les polymères fixants non ioniques sont choisis parmi les polymères non ioniques à base de vinylpyrrolidone, et dans laquelle l'alcanol inférieur en C₁-C₆ est l'éthanol et l'ester d'acide gras est un monocarboxytate d'alkyle en C₁-C₂₂.

2. Composition cosmétique selon l'une quelconque des revendications précédentes **caractérisée en ce que** le ou les polymères fixants non ioniques représentent généralement de 0,01 à 8%, de préférence de 0,1 à 3% en poids par rapport au poids total de la composition.

3. Composition cosmétique selon l'une quelconque des revendications précédentes **caractérisée en ce que** l'huile de vaseline représente de 3 à 20%, de préférence de 5 à 10% par rapport au poids total de la composition.

4. Composition cosmétique l'une quelconque des revendications précédentes **caractérisée en ce que** le ou les alcanols représentent de 5 à 75%, de préférence de 10 à 60%, mieux de 15 à 50% en poids par rapport au poids total de la composition.

5. Composition cosmétique selon l'une quelconque des revendications précédentes **caractérisée en ce que** le ou les esters d'acide gras représentent de 10 à 35%, mieux de 10 à 30% en poids par rapport au poids total de la composition.

6. Composition selon l'une quelconque des revendications précédentes **caractérisée en ce qu'**elle comprend au moins additif cosmétique choisi parmi les agents protecteurs des fibres capillaires, les vitamines ou provitamines, les parfums, les conservateurs, les séquestrants, les agents acidifiants, alcalinisants ainsi que leurs mélanges.

7. Composition selon la revendication 6 **caractérisée en ce que** les additifs cosmétiques sont présents dans l'invention à raison de 0,001% à 20%, de préférence de 0,1 à 5% en poids par rapport au poids total de la composition.

8. Composition selon l'une quelconque des revendications précédentes **caractérisée en ce qu'**elle contient au moins une silicone volatile ou non, linéaire ou cyclique.

9. Composition selon la revendication 8 **caractérisée en ce qu'**au moins une silicone est choisie parmi les polyorganosiloxanes

10. Composition selon la revendication 9 **caractérisée** en ce les polyorganosiloxanes sont choisis parmi les polyalkylsiloxanes, les polyarylsiloxanes, les polyalkylarylsiloxanes, les polyorganosiloxanes modifiés par des groupements organofonctionnels, ainsi que leurs mélanges.

11. Composition selon la revendication 10 **caractérisée en ce qu'**au moins un polyorganosiloxane est choisi parmi les polyorganosiloxanes comportant des groupements polyéthylèneoxy et/ou polypropylèneoxy.

12. Composition selon la revendication 11 **caractérisée en ce que** les groupements polyéthylèneoxy et/ou polypropylèneoxy comportent des groupements alkyle en C₆-C₂₄.

13. Composition selon l'une quelconque des revendications 8 à 12 **caractérisée en ce que** les silicones sont également utilisées sous forme soluble, dispersée, micro dispersée, d'émulsions, de nanoémulsions ou de micromulsions.

14. Composition selon l'une quelconque des revendications précédentes **caractérisée en ce qu'**elle comprend moins de 1% de silicone en poids par rapport au poids total de la composition.

15. Composition selon l'une quelconque des revendications précédentes **caractérisée en ce qu'** elle comprend au moins un agent protecteur des fibres capillaires choisi parmi les filtres UV organiques hydrosolubles, liposolubles ou insolubles dans l'eau, les agents antiradicalaires, les agents antioxydants, les vitamines, les provitamines ainsi que leurs mélanges.

16. Composition selon l'une quelconque des revendications précédentes **caractérisée en ce qu'**elle comprend un ou plusieurs solvants additionnels cosmétiquement acceptables choisis parmi les polyols tels que le glycérol, le propylène glycol, le pentanediol et l'alcool benzylique.

17. Composition selon la revendication 16 **caractérisée en ce que** le ou les solvants additionnels cosmétiquement acceptables selon l'invention représentent de 0,001 à 5%, de préférence de 0,1 à 1% par rapport au poids total de la composition.

18. Composition selon l'une quelconque des revendications précédentes **caractérisée en ce qu'**elle se présente sous forme d'un spray.

19. Composition selon l'une quelconque des revendications précédentes **caractérisée en ce qu'**elle se présente sous forme d'un spray aérosol et **en ce qu'**elle comprend au moins un agent propulseur choisi parmi les alcanes volatils tels que le n-butane, le propane, l'isobutane, le pentane, le gaz carbonique, le protoxyde d'azote, le diméthyléther, l'azote, l'air comprimé ou leurs mélanges.

20. Composition cosmétique selon la revendication 19 **caractérisée en ce que** l'agent propulseur choisi est l'isobutane.

21. Procédé de fabrication d'une composition cosmétique selon l'une quelconque des revendications précédentes **caractérisé en ce qu'**il comprend
a) La dissolution de l'huile de vaseline dans ledit au moins un ester d'acide gras ; et
b) l'addition de la solution obtenue à l'étape a) dans un mélange préalablement formé d'au moins un polymère fixant non ionique et d'éthanol, le polymère fixant non ionique étant un polymère à base de vinylpyrrolidone.

22. Procédé selon la revendication 21 **caractérisé en ce qu'**il comprend la pressurisation de la composition cosmétique dans un récipient au moyen d'un agent de propulsion.

23. Utilisation d'une composition selon l'une quelconque des revendications 1 à 20 pour apporter de la brillance à la chevelure.

24. Utilisation selon la revendication 23 **caractérisée en ce que** la chevelure traitée possède un toucher naturel.

## Patentansprüche

1. Wasserfreie kosmetische Zusammensetzung, **dadurch gekennzeichnet, dass** sie mindestens ein nichtionisches fixierendes Polymer, ein Vaselineöl, mindestens ein C₁-C₆-Niederalkanol und mindestens einen Fettsäureester umfasst, wobei die nichtionischen fixierenden Polymere aus nichtionischen Polymeren auf der Basis von Vinylpyrrolidon ausgewählt sind und wobei das C₁-C₆-Niederalkanol Ethanol ist und der Fettsäureester ein C₁-C₂₂-Alkylmonocarboxylat ist.

2. Kosmetische Zusammensetzung nach einem der vorhergehenden Ansprüche, **dadurch gekennzeichnet, dass** das oder die nichtionische(n) fixierende(n) Polymer(e) im Allgemeinen 0,01 bis 8 Gew.-%, vorzugsweise von 0,1 bis 3 Gew.-%, bezogen auf das Gesamtgewicht der Zusammensetzung, ausmachen.

3. Kosmetische Zusammensetzung nach einem der vorhergehenden Ansprüche, **dadurch gekennzeichnet, dass** das Vaselineöl 3 bis 20%, vorzugsweise 5 bis 10%, bezogen auf das Gesamtgewicht der Zusammensetzung, ausmacht.

4. Kosmetische Zusammensetzung nach einem der vorhergehenden Ansprüche, **dadurch gekennzeichnet, dass** das oder die Alkanol(e) 5 bis 75 Gew.-%, vorzugsweise 10 bis 60 Gew.-%, besser 15 bis 50 Gew.-%, bezogen auf das Gesamtgewicht der Zusammensetzung, ausmachen.

5. Kosmetische Zusammensetzung nach einem der vorhergehenden Ansprüche, **dadurch gekennzeichnet, dass** der oder die Fettsäureester 10 bis 35 Gew.-%, besser 10 bis 30 Gew.-%, bezogen auf das Gesamtgewicht der Zusammensetzung, ausmachen.

6. Zusammensetzung nach einem der vorhergehenden Ansprüche, **dadurch gekennzeichnet, dass** sie mindestens einen kosmetischen Zusatzstoff umfasst, der aus Schutzmitteln für Haarfasern, Vitaminen oder Provitaminen, Duftstoffen, Konservierungsmitteln, Sequestrierungsmitteln, Ansäuerungsmitteln, Alkalinisierungsmitteln sowie deren Gemischen ausgewählt ist.

7. Zusammensetzung nach Anspruch 6, **dadurch gekennzeichnet, dass** die kosmetischen Zusatzstoffe in der Erfindung zu 0,001 Gew.-% bis 20 Gew.-%, vorzugsweise zu 0,1 bis 5 Gew.-%, bezogen auf das Gesamtgewicht der Zusammensetzung, vorhanden sind.

8. Zusammensetzung nach einem der vorhergehenden Ansprüche, **dadurch gekennzeichnet, dass** sie mindestens ein flüchtiges oder nicht-flüchtiges, gerades oder zyklisches Silikon enthält.

9. Zusammensetzung nach Anspruch 8, **dadurch gekennzeichnet, dass** mindestens ein Silikon aus Polyorganosiloxanen ausgewählt ist.

10. Zusammensetzung nach Anspruch 9, **dadurch gekennzeichnet, dass** die Polyorganosiloxane aus Polyalkylsiloxanen, Polyarylsiloxanen, Polyalkylarylsiloxanen, mit organo-funktionellen Gruppen modifizierten Polyorganosiloxanen sowie deren Gemischen ausgewählt sind.

11. Zusammensetzung nach Anspruch 10, **dadurch gekennzeichnet, dass** mindestens ein Polyorganosiloxan aus Polyorganosiloxanen, die Polyethylenoxy- und/oder Polypropylenoxy-Gruppen tragen, ausgewählt ist.

12. Zusammensetzung nach Anspruch 11, **dadurch gekennzeichnet, dass** die Polyethylenoxy- und/oder Polypropylenoxy-Gruppen C₆-C₂₄-Alkylgruppen tragen.

13. Zusammensetzung nach einem der Ansprüche 8 bis 12, **dadurch gekennzeichnet, dass** die Silikone auch in löslicher, dispergierter, mikrodispergierter Form, in Form von Emulsionen, Nanoemulsionen oder Mikroemulsionen verwendet werden.

14. Zusammensetzung nach einem der vorhergehenden Ansprüche, **dadurch gekennzeichnet, dass** sie weniger als 1 Gew.-% Silikon, bezogen auf das Gesamtgewicht der Zusammensetzung, umfasst.

15. Zusammensetzung nach einem der vorhergehenden Ansprüche, **dadurch gekennzeichnet, dass** sie mindestens ein Schutzmittel für Haarfasern umfasst, das aus wasserlöslichen, fettlöslichen oder wasserunlöslichen organischen UV-Filtern, Radikalfängern, Antioxidantien, Vitaminen, Provitaminen sowie deren Gemischen ausgewählt ist.

16. Zusammensetzung nach einem der vorhergehenden Ansprüche, **dadurch gekennzeichnet, dass** sie ein oder mehrere zusätzliche, kosmetisch annehmbare Lösungsmittel umfasst, die aus Polyolen, wie Glycerin, Propylenglykol, Pentandiol und Benzylalkohol ausgewählt sind.

17. Zusammensetzung nach Anspruch 16, **dadurch gekennzeichnet, dass** das oder die zusätzliche(n) kosmetisch annehmbare(n) Lösungsmittel gemäß der Erfindung 0,001 bis 5 Gew.-%, vorzugsweise 0,1 bis 1%, bezogen auf das Gesamtgewicht der Zusammensetzung, ausmachen.

18. Zusammensetzung nach einem der vorhergehenden Ansprüche, **dadurch gekennzeichnet, dass** sie in Form eines Sprays vorliegt.

19. Zusammensetzung nach einem der vorhergehenden Ansprüche, **dadurch gekennzeichnet, dass** sie in Form eines Aerosolsprays vorliegt, und dadurch, dass sie mindestens ein Treibmittel umfasst, das aus flüchtigen Alkanen, wie n-Butan, Propan, Isobutan, Pentan, Kohlendioxidgas, Distickstoffoxid, Dimethylether, Stickstoff, Druckluft oder deren Gemischen ausgewählt ist.

20. Kosmetische Zusammensetzung nach Anspruch 19, **dadurch gekennzeichnet, dass** das ausgewählte Treibmittel Isobutan ist.

21. Verfahren zur Herstellung einer kosmetischen Zusammensetzung nach einem der vorhergehenden Ansprüche, **dadurch gekennzeichnet, dass** es Folgendes umfasst
a) Lösen von Vaselineöl in dem mindestens einen Fettsäureester und
b) Zugabe der in Schritt a) erhaltenen Lösung zu einem zuvor hergestellten Gemisch aus mindestens einem nichtionischen fixierenden Polymer und Ethanol, wobei das nichtionische fixierende Polymer ein Polymer auf der Basis von Vinylpyrrolidon ist.

22. Verfahren nach Anspruch 21, **dadurch gekennzeichnet, dass** es das unter Druck Setzen der kosmetischen Zusammensetzung in einem Behälter mithilfe eines Treibmittels umfasst.

23. Verwendung einer Zusammensetzung nach einem der Ansprüche 1 bis 20, um dem Kopfhaar Glanz zu verleihen.

24. Verwendung nach Anspruch 23, **dadurch gekennzeichnet, dass** das behandelte Kopfhaar sich natürlich anfühlt.

## Claims

1. Water-free cosmetic composition **characterized in that** it comprises at least one non-ionic fixing polymer, one liquid petroleum jelly, at least one C₁-C₆ lower alkanol, and at least one fatty acid ester, in which the non-ionic fixing polymers are chosen from vinylpyrolidone-based non-ionic polymers, and in which the C₁-C₆ lower alkanol is ethanol and the fatty acid ester is a C₁-C₂₂ alkyl monocarboxylate.

2. Cosmetic composition according to any one of the preceding claims, **characterized in that** the non-ionic fixing polymer(s) generally represent(s) from 0.01% to 8%, preferably from 0.1% to 3% by weight relative to the total weight of the composition.

3. Cosmetic composition according to either one of the preceding claims, **characterized in that** the liquid petroleum jelly represents from 3% to 20%, preferably from 5% to 10% relative to the total weight of the composition.

4. Cosmetic composition according to any one of the preceding claims, **characterized in that** the alkanol(s) represent (s) from 5% to 75%, preferably from 10% to 60%, better still from 15% to 50% by weight relative to the total weight of the composition.

5. Cosmetic composition according to any one of the preceding claims, **characterized in that** the fatty acid ester(s) represent(s) from 10% to 35%, better still from 10% to 30% by weight relative to the total weight of the composition.

6. Composition according to any one of the preceding claims, **characterized in that** it comprises at least one cosmetic additive chosen from agents for protecting hair fibres, vitamins or provitamins, fragrances, preservatives, sequestering agents, acidifying agents, basifying agents, and also mixtures thereof.

7. Composition according to Claim 6, **characterized in that** the cosmetic additives are present in the invention in a proportion of from 0.001% to 20%, preferably from 0.1% to 5% by weight relative to the total weight of the composition.

8. Composition according to any one of the preceding claims **characterized in that** it contains at least one linear or cyclic, volatile or non-volatile silicone.

9. Composition according to Claim 8, **characterized in that** at least one silicone is chosen from polyorganosiloxanes.

10. Composition according to Claim 9, **characterized in that** the polyorganosiloxanes are chosen from polyalkylsiloxanes, polyarylsiloxanes, polyalkylarylsiloxanes, polyorganosiloxanes modified with organofunctional groups, and also mixtures thereof.

11. Composition according to Claim 10, **characterized in that** at least one polyorganosiloxane is chosen from polyorganosiloxanes comprising polyethyleneoxy and/or polypropyleneoxy groups.

12. Composition according to Claim 11, **characterized in that** the polyethyleneoxy and/or polypropyleneoxy groups comprise C₆-C₂₄ alkyl groups.

13. Composition according to any one of Claims 8 to 12, **characterized in that** the silicones are also used in soluble, dispersed, microdispersed, emulsion, nanoemulsion or microemulsion form.

14. Composition according to any one of the preceding claims, **characterized in that** it comprises less than 1% of silicone by weight relative to the total weight of the composition.

15. Composition according to any one of the preceding claims, **characterized in that** it comprises at least one agent for protecting hair fibres chosen from watersoluble, liposoluble or water-insoluble organic UV-screening agents, free-radical scavengers, antioxidants, vitamins, provitamins, and also mixtures thereof.

16. Composition according to any one of the preceding claims, **characterized in that** it comprises one or more cosmetically acceptable additional solvents chosen from polyols such as glycerol, propylene glycol, pentanediol and benzyl alcohol.

17. Composition according to Claim 16, **characterized in that** the cosmetically acceptable additional solvent(s) according to the invention represent(s) from 0.001% to 5%, preferably 0.1% to 1% relative to the total weight of the composition.

18. Composition according to any one of the preceding claims, **characterized in that** it is in the form of a spray.

19. Composition according to any one of the preceding claims, **characterized in that** it is in the form of an aerosol spray and **in that** it comprises at least one propellant chosen from volatile alkanes such as n-butane, propane, isobutane, pentane, carbon dioxide, nitrous oxide, dimethyl ether, nitrogen, compressed air or mixtures thereof.

20. Cosmetic composition according to Claim 19, **characterized in that** the propellant chosen is isobutane.

21. Process for producing a cosmetic composition according to any one of the preceding claims, **characterized in that** it comprises
a) dissolving the liquid petroleum jelly in said at least one fatty acid ester; and
b) adding the solution obtained in step a) to a preformed mixture of at least one non-ionic fixing polymer and ethanol, the non-ionic fixing polymer being a vinyl pyrrolidone-based polymer.

22. Process according to Claim 21, **characterized in that** it comprises pressurizing the cosmetic composition in a container by means of a propellant.

23. Use of a composition according to any one of Claims 1 to 20, for giving a sheen to the head of hair.

24. Use according to Claim 23 **characterized in that** the head of hair treated has a natural feel.
